# EUROPÄISCHE PATENTSCHRIFT

(11) **EP 2 278 942 B1**
(45) Veröffentlichungstag und Bekanntmachung des Hinweises auf die Patenterteilung: **06.01.2016**
(21) Anmeldenummer: 09749605.3
(22) Anmeldetag: 19.05.2009
(51) Int. Cl.: A61F 2/68, A61F 2/64

(54) **Orthopädische Einrichtung mit einem Gelenk sowie Verfahren zur Steuerung dieser orthopädischen Einrichtung**
Orthopedic device comprising a joint and method for controlling said orthopedic device
Dispositif orthopédique pourvu d'une articulation et procédé pour commander ce dispositif orthopédique

(30) Priorität: 20.05.2008 DE 102008024747
(43) Veröffentlichungstag der Anmeldung: 02.02.2011
(73) Patentinhaber: Otto Bock Healthcare Products GmbH, 1070 Wien (AT)
(72) Erfinder: KAMPAS, Philipp, 1030 Wien (AT); PAWLIK, Roland, 1130 Wien (AT)
(74) Vertreter: Stornebel, Kai
(86) Internationale Anmeldenummer: PCT/EP2009/003562
(87) Internationale Veröffentlichungsnummer: WO 2009/141115

(56) Entgegenhaltungen:
- WO-A1-01/43669
- WO-A1-2008/080231
- WO-A2-03/086245
- WO-A2-2007/027668
- WO-A2-2008/044207
- WO-A2-2008/048658
- GB-A- 2 328 160
- US-A- 6 113 642

## Beschreibung

Die Erfindung betrifft eine orthopädische Einrichtung mit einem Gelenk, die ein Oberteil und ein schwenkbar daran gelagertes Unterteil aufweist, wobei an dem Oberteil obere Anschlussmittel zur Befestigung an einer Gliedmaße angeordnet sind, sowie einer Verriegelungseinrichtung, die eine Beugebewegung des Oberteils relativ zu dem Unterteil verhindert, wobei die Verriegelungseinrichtung aktiv durch den Nutzer der orthopädischen Einrichtung betätigbar ausgestaltet ist. Die Erfindung betrifft ebenfalls ein Verfahren zur Steuerung einer orthopädischen Einrichtung. Insbesondere werden solche orthopädischen Einrichtungen als Orthesen oder Prothesen eingesetzt, bevorzugt als Kniegelenksorthesen oder Prothesenkniegelenke.

Orthopädische Einrichtungen mit zwei relativ zueinander beweglichen Teilen und einer dazwischen angeordneten Verriegelungseinrichtung zum Verriegeln der beiden Teile in einer vorbestimmten relativen Position sowie zum Entriegeln der Teile zur Freigabe der Bewegung der Teile zueinander werden für zahlreiche Anwendungen eingesetzt, um am menschlichen Körper vorübergehend oder dauerhaft vorhandene Schwächen zu kompensieren oder sonst nicht ausübbare Funktionen zu ermöglichen. Bei einer Versorgung von Patienten mit einem niedrigen Mobilitätsgrad, also Patienten, die sich vorwiegend im Innenbereich aufhalten und mitunter in ihren muskulären und motorischen Leistungsfähigkeiten eingeschränkt sind, also bei sogenannten geriatrischen Patienten, werden häufig orthopädische Einrichtungen mit einem Sperrgelenk eingerichtet, wobei solche Vorrichtungen insbesondere zur Unterstützung der Beinfunktion eingesetzt werden. So können beispielsweise Knieorthesen oder Prothesenkniegelenke als Sperrgelenke eingesetzt werden, die bei völliger Streckung in Folge eines Sperrmechanismusses automatisch verriegelt werden und für eine Beugung manuell entriegelt werden müssen. Für Kniegelenke stellt ein gesperrtes Kniegelenk, sei es als Prothese oder Orthese, die sicherste Konfiguration für Stehen und Gehen dar. Sicherheit gegen Stürze ist insbesondere bei der Versorgung geriatrischer Patienten ein wichtiges Ziel, wobei die Entriegelung überwiegend durch Ziehen an einem Zugseil erfolgt, welches von dem Kniegelenk hinauf zu dem Oberschenkel verlegt wird, so dass das Zugseil im Stehen gut erreicht werden kann. Das Stehen und Gehen erfolgt also mit einem steifen Kniegelenk, das Sitzen bei einem gebeugten, freibeweglichen Kniegelenk, sofern das Kniegelenk im Sitzen nicht voll gestreckt wird.

Die DE 103 11 187 A1 beschreibt ein orthopädietechnisches Hilfsmittel mit einer Verriegelungsvorrichtung zum Verriegeln und Entriegeln zweier beweglich zueinander gelagerten Teile, wobei die Verriegelungsvorrichtung von einem Steuermodul aus elektromechanisch betätigbar ist und ein Betätigungssignal von einer Betätigungseinheit aus drahtlos auf das Steuermodul übertragen wird. Die Betätigungseinheit kann in eine Gehhilfe integriert sein, so dass die Verriegelungsvorrichtung mittels einer Fernbedienung entriegelt werden kann.

Die DE 103 51 916 A1 beschreibt ein Prothesenkniegelenk für geriatrische Patienten, das mit einer hydraulischen Dämpfereinheit versehen ist, die als Verriegelungseinrichtung betreibbar ist. Innerhalb eines festgelegten Winkelbereiches wird eine Beugung der Gelenkeinrichtung blockiert, wobei außerhalb des festlegbaren Winkelbereiches das Unterteil in Beugerichtung frei verschwenkbar ist. Eine Streckung des Unterteils soll jederzeit möglich sein. Ein solches Prothesenkniegelenk dient als Aufsteh- und Hinsetzhilfe.

Die EP 1 237 513 B1 betrifft eine die Existenz oder Funktion einer Gliedmaße ersetzende Unterstützungsvorrichtung, die einen Sensor umfasst, wobei der Sensor die Position eines mit einem Gelenk verbundenen Elements relativ zu einer fixen Linie detektiert, wobei der Sensor mit einer Steuerungsvorrichtung gekoppelt ist, die zur Steuerung des Gelenks auf Basis der Positionsdaten des Sensors vorgesehen ist. Die Vorrichtung zur Steuerung des Kniegelenks ist in ihrer einfachsten Form eine Verriegelungs- oder Bremsvorrichtung.

Die WO 03/086245 A2 beschreibt eine Verriegelungseinrichtung in Gestalt eines Elektromagneten, der eine formschlüssige Verriegelung eines Gelenkes bewirkt, wenn keine ausreichende elektrische Energie vorhanden ist. Die als Sicherheitseinrichtung ausgebildete Verriegelungseinrichtung ist als automatische Sperreinrichtung ausgebildet, eine aktive Betätigung der Verriegelungseinrichtung kann nicht erfolgen.

Die WO 02/43669 A1 beschreibt eine Verriegelungseinrichtung in Gestalt eines Magnetventils oder einer Hydraulikpumpe, die über einen Schwerkraftsensor automatisch entriegelt oder verriegelt wird. Über ein manuelles Steuerungsmittel in Gestalt eines Druckknopfes kann die Verriegelungseinrichtung manuell entriegelt werden.

Die WO 2008/044207 A2 beschreibt eine Immobilisierungseinrichtung mit einer magneto-rheologischen Flüssigkeit, über die auf der Grundlage eines Bewegungssensors ein Gelenk gesperrt wird.

Die WO 2008/080231 A1 beschreibt eine Antriebseinrichtung für ein prothetisches oder ortethisches Gelenk mit einem Verriegelungsmechanismus. Der Verriegelungsmechanismus erstreckt sich außerhalb des Gelenkabschnittes und befindet sich in operativer Verbindung zu dem Aktuator, um wahlweise ein Verriegeln und Entriegeln des Aktuators zu bewirken.

Die GB 2 328 160 A betrifft eine hydropneumatische Prothese einer unteren Extremität mit einer Zylinderanordnung zwischen einem Urteil und einem gelenkig daran befestigten Unterteil, wobei die Zylinderanordnung aus einer hydraulischen Kolben-Zylindereinheit und einer hydraulischen Kolben-Zylindereinheit besteht. In Abhängigkeit von Sensorsignalen wird automatisch der Flexions- und Extensionswiderstand entsprechend dem Aktivitätsmodus des Patienten angepasst. Ein Verriegelungsmodus kann auch freiwillig durch den Nutzer ausgewählt und aufgehoben werden, beispielsweise durch wiederholtes Belasten und Entlasten der Gliedmaße in einer besonderen Sequenz, vorausgesetzt, dass das Knie gestreckt ist. Zur Deaktivierung kann auch ein Schalter vorgesehen sein. Über einen Flexionsbereich bis 20° wird die Dämpfung überwiegend von dem hydraulischen Teil gesteuert, die darüber hinausgehende Flexion wird von dem pneumatischen Teil gesteuert. Die hydraulische Dämpfereinheit verfügt über einen wesentlich höheren Widerstand als die pneumatische Dämpfereinheit vergleichbarer Größe.

Die US 6,113,642 A betrifft eine computergesteuerte hydraulische Widerstandseinrichtung für ein Prothesenkniegelenk mit einem Oberteil und einem verschwenkbar gelagerten Unterteil. Drucksensoren sind auf beiden Seiten des Hydraulikkolbens angeordnet, um automatisch Veränderungen in der Vorrichtung und in der Viskosität der Hydraulikflüssigkeit auszugleichen. Sowohl Belastungssensoren als auch Winkelsensoren sind vorgesehen, um über Ventile den hydraulischen Widerstand zu steuern. Ziel ist es, eine automatische Anpassung des hydraulischen Widerstandes bei einer Kniebeugung vorzunehmen, so dass ein natürliches Gangbild bereitgestellt werden kann.

Aufgabe der vorliegenden Erfindung ist es, eine orthopädische Einrichtung und ein Verfahren zum Steuern einer orthopädischen Einrichtung bereitzustellen, mit denen bei ausreichender Sicherheit während des Gehens und Stehens ein höheres Maß an Bequemlichkeit erreicht werden kann.

Erfindungsgemäß wird diese Aufgabe durch eine orthopädische Einrichtung mit den Merkmalen des Hauptanspruches sowie ein Verfahren zur Steuerung einer solchen orthopädischen Einrichtung mit den Merkmalen des Anspruchs 10 gelöst. Vorteilhafte Ausgestaltungen und Weiterbildungen der Erfindung sind in den Unteransprüchen beschrieben.

Die erfindungsgemäße orthopädische Einrichtung mit einem Gelenk, die ein Oberteil und ein schwenkbar daran gelagertes Unterteil aufweist, wobei an dem Oberteil obere Anschlussmittel zur Festlegung an einer Gliedmaße angeordnet sind, sowie einer Verriegelungseinrichtung, die eine Beugebewegung des Oberteils relativ zu dem Unterteil verhindert, wobei die Verriegelungseinrichtung aktiv durch den Nutzer der orthopädischen Einrichtung betätigbar ausgestaltet ist, sieht vor, dass eine Steuerungseinrichtung der Verriegelungseinrichtung zugeordnet ist, die mit zumindest einem Sensor, der an der Einrichtung angebracht ist, verbunden ist und in Abhängigkeit von dem Sensorsignal die Verriegelungseinrichtung automatisch entriegelt oder sperrt. Während es bei herkömmlichen orthopädischen Einrichtungen, insbesondere bei Prothesenkniegelenken oder Kniegelenksorthesen notwendig ist, zum Beugen des Gelenks aktiv eine Verriegelung zu lösen, sieht die erfindungsgemäße orthopädische Einrichtung vor, dass zusätzlich zu einer willkürlichen Ver- und Entriegelung der Gelenkeinrichtung diese auch unwillkürlich in Abhängigkeit von Sensorinformationen von Sensoren, die an der orthopädischen Einrichtung angeordnet ist, entriegelt oder gesperrt wird. Neben einer willkürlichen Aktivierung der Ver- und Entriegelung ist es möglich, ein automatisches Lösen einer Sperre oder ein Verringern eines Widerstandes des Gelenks in Abhängigkeit von der Lage des Oberteils oder des Unterteils oder anderen Parametern vorzunehmen, so dass gerade bei Nutzern mit eingeschränkten motorischen Fähigkeiten eine Erleichterung der Benutzung auftritt. Im Hinblick auf Prothesenkniegelenke ist es somit möglich, dass sich ein Nutzer mit einem verriegelten Gelenk hinsetzen kann, ohne die Verriegelungseinrichtung aktiv entsperren zu müssen, wobei bel Erreichen einer vorgegebenen Orientierung des Oberteils und/oder des Unterteils das Gelenk automatisch entsperrt wird, so dass eine freie Beweglichkeit im Sitzen gegeben ist. Weiterhin wird verhindert, dass im Sitzen bei einer vollständigen Streckung des Beines das Gelenk verriegelt, was in der sitzenden Position des Prothesenträgers hinderlich ist. Eine entsprechende Anwendung ist auch auf die Gelenksorthesen möglich, wobei hier an dem Unterteil Befestigungseinrichtungen zur Befestigung an einer Gliedmaße, beispielsweise an dem Unterschenkel oder dem Unterarm, vorgesehen sind. Bei einer Ausgestaltung der orthopädischen Einrichtung als ein Prothesengelenk ist an dem Unterteil die Anbringung weiterer orthopädischer oder prothetischer Komponenten vorgesehen.

Die Ausbildung der Verriegelungseinrichtung als Hydraulikdämpfer kann auch so gestaltet sein, dass der Hydraulikdämpfer vor einem Streckanschlag bis zu einem festgelegten Beugewinkel eine gedämpfte Beugung ermöglicht, während ab Erreichen des festgelegten Beugewinkels die weitere Beugung gesperrt ist. Dies kann über eine mechanische Steuerung erfolgen, die ein Spiel innerhalb der Verriegelungseinrichtung ermöglicht, beispielsweise indem über einen Bereich von 5° bis 10 ° ab dem Streckanschlag eine Bypassleitung offen gehalten wird, die nach Erreichen dieses Beugewinkels durch eine Steuerscheibe oder dergleichen geschlossen wird. Alternativ dazu kann die Sperrung Ober die Steuereinrichtung erfolgen.

Eine Weiterbildung der Erfindung sieht vor, dass der Sensor als ein Lagesensor zur Erfassung der Orientierung des Oberteils und/oder Unterteils im Raum, als ein Winkelsensor zur Erfassung des Winkels zwischen dem Oberteil und dem Unterteil, als Kraftsensor zur Erfassung einer in der orthopädischen Einrichtung wirksamen Kraft und/oder als ein Momentensensor zur Erfassung eines in der orthopädischen Einrichtung wirksamen Momentes ausgebildet und mit der Steuereinrichtung gekoppelt ist. Beispielsweise kann bei der Erkennung der Winkeländerung zwischen Oberteil und Unterteil aus der Drehrichtung in Verbindung mit der absoluten Lage des Oberteils und des Unterteils darauf geschlossen werden, ob ein Patient aufstehen möchte, so dass beim Aufstehen automatisch schon vor Erreichen einer vollen Kniestreckung das Gelenk automatisch gesperrt werden kann. Die Sperrung kann auch nur in einer Richtung erfolgen, so dass die Streckung stets möglich ist, jedoch eine Beugung bereits in einer ersten Phase des Aufstehens unterbunden werden kann, um ein unbeabsichtigtes Zurückfallen des Patienten beim Aufstehen zu vermeiden. Damit ist es beispielsweise möglich, dass ein Kniegelenk während des Aufstehens gesperrt wird, wenn die Streckung nur annähernd erreicht wird, beispielsweise 15° vor Erreichen eines Streckanschlages, wenn der Unterschenkel näherungsweise vertikal ausgerichtet ist. Neben der Berücksichtigung des Winkels zwischen dem Oberteil und dem Unterteil kann ein Sensor zur Erfassung der Orientierung des Oberteils und/oder Unterteils im Raum vorgesehen sein, um allein oder in Verbindung mit den Winkelsensordaten eine automatische Entriegelung oder Sperrung der Verriegelungseinrichtung bewirken zu können. Bei einer waagerechten Orientierung des Oberteils bei einer Ausgestaltung als ein Prothesenkniegelenk kann so bei einer im Wesentlichen waagerechten Orientierung des Oberteils bei einem gleichzeitigen angenäherten rechten Winkel zwischen Oberteil und Unterteil darauf geschlossen werden, dass sich der Nutzer in einer sitzenden Stellung befindet, so dass automatisch eine Entriegelung stattfindet. Ebenfalls ist es möglich, dass ein Kraft- oder Momentensensor zur Ermittlung der in der orthopädischen Einrichtung wirksamen Kraft oder des in der orthopädischen Einrichtung wirksamen Momentes ausgebildet ist, wobei die Kraft- und/oder Momentensensoren auch in Ergänzung zu dem Winkelsensor oder dem Lagesensor eingesetzt werden können. In dem Unterteil ist bevorzugt ein Kraftsensor zur Erfassung einer in dem Unterteil wirkenden Kraft, insbesondere Axialkraft, angeordnet, der mit der Steuereinrichtung verbunden ist. Auf diese Weise kann erkannt werden, ob das Unterteil belastet ist, beispielsweise, ob ein Patient steht und die orthopädische Einrichtung, insbesondere die Prothese, belastet oder nicht. So kann im Sitzen bei einer nicht vorhandenen oder nur sehr geringen Axialbelastung bei vertikaler Orientierung des Unterteils, eine Sperrung unterbleiben, während bei einer axialen Belastung, beispielsweise während des Stehens oder des Aufstehens, eine Sperrung in Beugerichtung eingeleitet werden kann, um ein Rückfallen des Patienten zu verhindern. Dadurch wird auch verhindert, dass das Gelenk während des Sitzens verriegelt, wenn das Bein oder die Prothese vollständig gestreckt wird und gleichzeitig erkannt wird, dass keine Axiallast in dem Unterteil wirksam ist. Ein Gelenkmomentsensor zur Erfassung des wirksamen Momentes in dem Gelenk zwischen Oberteil und Unterteil kann vorgesehen sein, wobei der Gelenkmomentsensor ebenfalls mit der Steuereinrichtung verbunden ist. Über den Gelenkmomentsensor können weitere Informationen über die wirksamen Kräfte innerhalb der orthopädischen Einrichtung ermittelt werden, so dass die Steuerung präziser erfolgen kann.

Die orthopädische Einrichtung ist bevorzugt als ein Orthesenkniegelenk oder Prothesenkniegelenk ausgebildet, jedoch ist auch grundsätzlich die Anwendung als beispielsweise Ellenbogenorthese oder Unterarmprothese vorgesehen.

Bevorzugt ist die Verriegelungseinrichtung ein Hydraulikdämpfer, der zumindest ein über einen Aktuator schaltbares Ventil oder eine über einen Aktuator schaltbare Drosselstelle aufweist, wobei der Aktuator mit der Steuereinrichtung verbunden ist. Dadurch ist es möglich, die hydraulische Dämpfung zu erhöhen oder zu verringern, um ein Gelenk zu verriegeln, wenn ein entsprechendes Ventil vollständig geschlossen wird oder einen gewünschten Dämpfungswiderstand bereitzustellen, wenn ein Ventil oder eine Drosselstelle in einem bestimmten Maße geöffnet oder geschlossen wird.

Eine Weiterbildung der Erfindung sieht vor, dass der Steuereinrichtung ein Zeitschaltglled zugeordnet ist, das eine Entriegelung oder Verriegelung bzw. Sperrung der Verriegelungseinrichtung erst nach einem entsprechenden Sensorsignal verzögert. Durch dieses Zeitschaltglied ist es möglich, eine Vielzahl von Steuerungsvarianten zu ermöglichen, beispielsweise ein zeitgesteuertes Offenhalten nach einer automatischen Entriegelung oder eine zeitgesteuerte Wiederverriegelung nach der Steuersignalentriegelung. Ebenfalls ist es möglich, dass nach einer willkürlichen Entriegelung eine erneute Verriegelung für eine gewisse Zelt unmöglich ist, so dass der Patient ausreichend Zeit hat, sich nach einer Entriegelung beispielsweise zusätzlichen Halt zu verschaffen und sich hinzusetzen. Ebenfalls kann nach einer willkürlichen Entriegelung eine neuerliche Verriegelung nach Ablauf einer gewissen Zelt automatisch erfolgen, sofern das Sensorsignal bzw. die Sensorsignale eine Entriegelung nicht erkennen lassen, also eine Benutzung nach der Entriegelung, so dass angenommen werden kann, dass der Nutzer die Entriegelung vergessen hat. Daher wird zur Sicherheit eine automatische Verriegelung eingeleitet.

Der orthopädischen Einrichtung kann ein Dämpfungselement zugeordnet sein, das dergestalt auf den Aktuator einwirkt, dass eine Entriegelung kontinuierlich erfolgt, also dass beim Entriegeln die Ventile oder Drosselstellen nur langsam geöffnet werden und nicht schlagartig, so dass eine Entriegelung nicht unmittelbar erfolgt, was für die meisten Nutzer eine Verunsicherung darstellen würde.

Zur Erhöhung des Komforts ist es vorgesehen, dass die Steuereinrichtung auch fernbedienbar ausgebildet Ist, um neben einer automatischen Steuerung des Gelenks auch eine individuelle Steuerung vornehmen zu können. Dadurch wird gewährleistet, dass der Nutzer der orthopädischen Einrichtung jederzeit den Verriegelungszustand des Gelenkes aktiv beeinflussen kann.

Ein Signalgeber kann an der Einrichtung angeordnet sein, der ein Öffnen der Verriegelungseinrichtung anzeigt oder ankündigt, so dass der Nutzer der orthopädischen Einrichtung beim Öffnen. also Entriegeln des Gelenks oder kurz davor, informiert wird, dass die sichere Verriegelung aufgehoben ist oder aufgehoben werden wird.

Ein Verfahren zur Steuerung einer wie oben beschriebenen orthopädischen Einrichtung sieht vor, dass die Verriegelungseinrichtung in Abhängigkeit von einer ermittelten Orientierung des Oberteils und/oder des Unterteils im Raum freigeschaltet oder gesperrt wird. insbesondere wird bei einer im Wesentlichen horizontalen Ausrichtung des Unterteils die Verriegelungseinrichtung entriegelt, da dann angenommen wird, dass bei einer Ausgestaltung der orthopädischen Einrichtung als eine Knieorthese oder als ein Prathesenkniegelenk der Patient sitzt und das Unterteil bzw. der Unterschenkel frei beweglich sein soll.

Weiterhin ist es vorgesehen, dass das Steuerungsverfahren während einer Streckbewegung des Gelenkes bei Überschreiten eines festgelegten Gelenkwinkels eine Beugebewegung verriegelt, während eine weitere Streckbewegung weiterhin möglich ist, dies ist insbesondere für Prothesen oder Orthesen der unteren Extremitäten vorteilhaft, grundsätzlich ist es jedoch auch möglich, die Bewegungsrichtungen umzukehren, wenn als sichere Stellung die gebeugte anzusehen ist.

Ebenfalls kann der Winkel zwischen Oberteil und Unterteil ermittelt und die Verriegelungseinrichtung in Abhängigkeit von dem ermittelten Winkel entriegelt oder gesperrt werden, wobei eine in dem Unterteil wirksame Kraft ermittelt und in Abhängigkeit von der ermittelten Kraft die Verriegelungseinrichtung entriegelt oder gesperrt werden kann. Insbesondere für Prothesenkniegelenke ist es vorteilhaft, wenn ausgehend von einem Streckanschlag über einen festgelegten Beugebereich In der Verriegelungseinrichtung ein Dämpfungswiderstand bereitstellt wird, der eine gedämpfte Beugung ermöglicht und bei Überschreiten des festgelegten Beugewinkels die Verriegelungseinrichtung gesperrt wird. Hierdurch ist es möglich, dass mit einem leicht gebeugten Prothesenkniegelenk gestanden werden kann, was dem natürlichen Erscheinungsbild besser entspricht. Ebenfalls kann vorgesehen sein, dass vor dem Freischalten der Verriegelungseinrichtung ein Warnsignal ausgegeben wird, so dass eine Rückmeldung des Gelenkes entweder akustisch, optisch oder taktil erfolgt, um den Patienten darüber zu informieren, dass ein tatsächliches Entsperren erfolgt bzw. erfolgt ist. Die Entsperrung kann kontinuierlich erfolgen, insbesondere bei einer Ausgestaltung der Verriegelungseinrichtung als ein Hydraulikdämpfer, Indem die entsprechenden Ventile oder Drosselstellen langsam geöffnet werden. Über einen Zeltschalter kann verhindert werden, dass sich das Gelenk nach dem Entriegeln sofort wieder sperrt, so dass ein Patient Zeit hat, sich auf das Hinsetzen bzw. das Beugen des Gelenkes vorzubereiten. Nach einer vorgegebenen Zeit wird eine automatische Verriegelung der Verriegelungseinrichtung durchgeführt, um die gelöste Stellung des Gelenkes nicht über einen längeren Zeitraum vorliegen zu haben, da der Fall auftreten kann, dass zwar eine aktive Auslösung und Entsperren des Gelenkes erfolgt, dieses jedoch von dem Patienten wieder vergessen wird. Dadurch wird gewährleistet, dass es nach einer vorbestimmten Zeitdauer, wenn das Gelenk dann nicht gebeugt wird, automatisch wieder selbst verriegelt wird, damit sich das Gelenk wieder im gesicherten Zustand befindet.

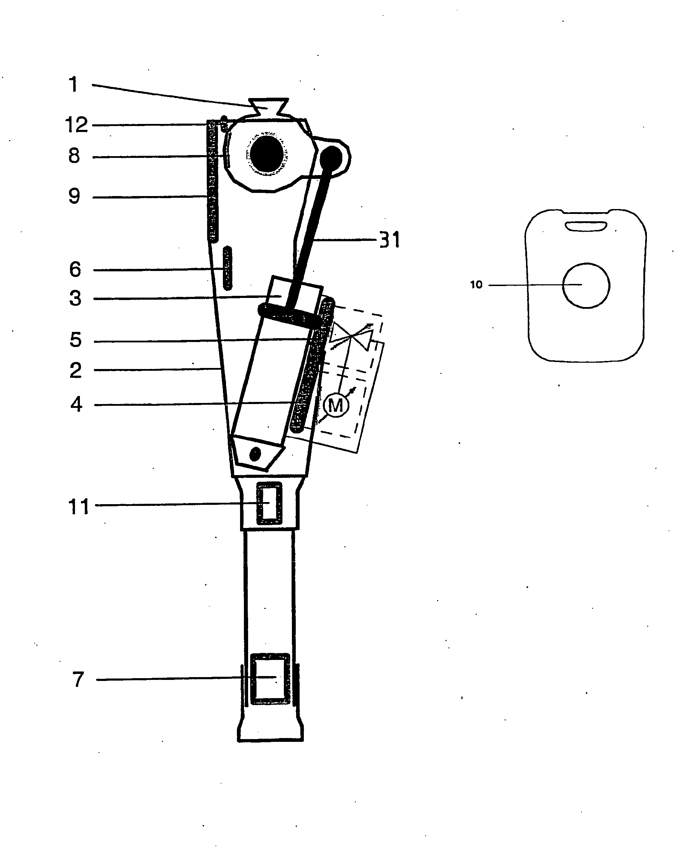

Nachfolgend wird ein Ausführungsbeispiel der Erfindung anhand der beigefügten Figur näher erläutert. Die einzige Figur zeigt eine schematische Darstellung einer orthopädischen Einrichtung in Gestalt eines Prothesenkniegelenkes.

Die Figur zeigt in einer schematischen Darstellung eine orthopädische Einrichtung in Gestalt eines Prothesenkniegelenkes mit einem Gelenkoberteil 1 mit Anschlussmitteln für einen Prothesenschaft und einem Gelenkunterteil 2, das schwenkbar an dem Gelenkoberteil 1 befestigt ist. Eine Verriegelungseinrichtung 3 in Gestalt eines Hydraulikdämpfers ist an dem Unterteil 2 befestigt und über eine Kolbenstange 31 mit dem Oberteil gekoppelt. Der Hydraulikdämpfer 3 ist mit einem Aktuator 4 versehen, der ein Ventil 5, das dem Hydraulikdämpfer 3 zugeordnet ist, öffnet oder schließt, um die Verriegelungseinrichtung 3 entweder zu sperren oder zu entriegeln. Das Sperren oder Entriegeln erfolgt durch Öffnen oder Schließen des Ventils 5, wobei neben einer abrupten Öffnung oder Schließung des Ventils 5 auch ein jeweils länger dauernder Übergang zwischen den Zuständen "Offen" und "Geschlossen" realisiert werden kann.

In dem Unterteil 2 sind mehrere Sensoren 6, 7, 8 angeordnet, die mit einer Steuereinrichtung 9 gekoppelt sind, die auf der Grundlage der Sensorsignale den Aktuator 4 in Gestalt eines Motors oder Schaltelementes ansteuert. Ein Lagesensor 6 ist in dem Unterteil 2 angeordnet, um die Orientierung des Unterteils 2 in dem Raum zu erfassen. Hierbei wird bevorzugt die Orientierung relativ zu der Schwerkraft ermittelt, so dass sich die Lage des Unterteils 2 leicht bestimmen lässt. Neben dem Lagesensor 6 ist ein Kraft- und Momentensensor 7 in dem distalen Bereich des Unterteils 2 angeordnet, um wirksame Kräfte und Momente innerhalb des Unterteils 2 erfassen zu können, beispielsweise um Axialkräfte, die in dem Unterteil 2 wirken, oder Biegemomente, die beim Gehen oder Stehen auftreten, erfassen zu können. Im Bereich des Oberteils 1 ist ein Kniewinkelsensor 8 angeordnet, der die relative Position des Oberteils 1 und der sich daran befindlichen Anschlusselemente an eine menschliche Gliedmaße zu dem Unterteil 2 ermittelt. Aus den Daten der Sensoren 6, 7, 8 wird dann in der Steuereinrichtung 9 errechnet, ob die Verriegelungseinrichtung 3 entsperrt, verriegelt oder in einen Dämpfungsmodus geschaltet wird, der der detektierten Situation am besten angepasst ist. So kann bei einem erwarteten Aufstehen eine Streckung des Oberteils 1 relativ zu dem Unterteil 2 ermöglicht, ein Rückfallen jedoch verhindert werden, indem eine entsprechende Steuerschaltung bei einem sich umkehrenden Kniewinkel und/oder einer entsprechenden Kraft innerhalb des Unterteils gesperrt wird.

Neben der Prothese ist eine Fernbedienung 10 mit einem Taster für die Eingabe eines oder mehrerer Steuerbefehle dargestellt, so dass neben einer automatischen Entriegelung oder Sperrung der Verriegelungseinrichtung 3 diese auch willkürlich von einem Nutzer gesperrt oder entriegelt werden kann. Eine Ausgestaltung der Betätigungseinrichtung 10 als Fernbedienung hat den Vorteil, dass diese an jedem beliebigen Ort positioniert werden kann, so dass die Verriegelungseinrichtung 3 unabhängig von der gegenwärtigen Position und von den Fähigkeiten des Nutzers, gesperrt oder entriegelt werden kann.

Neben einem akustischen Signalgeber 11, der ebenfalls in dem Unterteil 2 angeordnet ist, um ein Entriegeln oder ein bevorstehendes Entriegeln anzuzeigen, kann auch ein Vibrationssignalgeber 12 an der Prothese angeordnet sein, damit auch in einem lauteren Umfeld oder bei einer eingeschränkten Hörfähigkeit der Prothesennutzer darüber informiert wird, dass ein Auslösen ausgeführt wird oder unmittelbar bevorsteht.

Mit der dargestellten Prothese ist es möglich, eine Entriegelung auch unter Last durchzuführen, ebenfalls ist es möglich, unter Last eine Verriegelung zu bewirken, so dass jederzeit eine Beugebewegung gesperrt werde kann, um dem Nutzer ein Höchstmaß an Sicherheit zu gewährleisten. Neben einer Fernbedienung 10 kann eine entsprechende Betätigungseinrichtung auch an der orthopädischen Einrichtung selbst angeordnet sein. Neben einer Ausgestaltung als Prothesenkniegelenk kann die orthopädische Einrichtung auch als eine Kniegelenksorthese oder eine Prothese oder Orthese für obere Extremitäten vorgesehen sein.

## Patentansprüche

1. Orthopädische Einrichtung mit einem Gelenk, die ein Oberteil (1) und ein schwenkbar daran gelagertes Unterteil (2) aufweist, wobei an dem Oberteil (1) obere Anschlussmittel zur Befestigung an einer Gliedmaße angeordnet sind, sowie einer Verriegelungseinrichtung (3), die eine Beugebewegung des Oberteils (1) relativ zu dem Unterteil (2) verhindert, wobei die Verriegelungseinrichtung (3) aktiv durch den Nutzer der orthopädischen Einrichtung betätigbar ausgestaltet und eine Steuereinrichtung (9) der Verriegelungseinrichtung (3) zugeordnet ist, die mit zumindest einem Sensor (6, 7, 8), der an der Einrichtung angebracht ist, verbunden ist und in Abhängigkeit von dem Sensorsignal die Verriegelungseinrichtung (3) automatisch entriegelt oder sperrt, wobei die Verriegelungseinrichtung (3) als Hydraulikdämpfer ausgebildet ist, **dadurch gekennzeichnet, dass** der Hydraulikdämpfer von einem Streckanschlag bis zu einem festgelegten Beugewinkel eine gedämpfte Beugung ermöglicht und ab dem Erreichen des festgelegten Beugewinkels die weitere Beugung sperrt.

2. Orthopädische Einrichtung nach Anspruch 1, **dadurch gekennzeichnet, dass** der Sensor als ein Lagesensor (6) zur Erfassung der Orientierung des Oberteiles (1) und/oder Unterteiles (2) im Raum, als ein Winkelsensor (8) zur Erfassung des Winkels zwischen dem Oberteil (1) und dem Unterteil (2), als Kraftsensor (7) zur Erfassung einer in der orthopädischen Einrichtung wirksamen Kraft und/oder als ein Momentensensor (7) zur Erfassung eines in der orthopädischen Einrichtung wirksamen Momentes ausgebildet und mit der Steuereinrichtung (9) gekoppelt ist.

3. Orthopädische Einrichtung nach Anspruch 1 oder 2, **dadurch gekennzeichnet, dass** die orthopädische Einrichtung als Orthesenkniegelenk oder Prothesenkniegelenk ausgebildet ist.

4. Orthopädische Einrichtung nach einem der voranstehenden Ansprüche, **dadurch gekennzeichnet, dass** die Verriegelungseinrichtung (3) ein Hydraulikdämpfer ist, der zumindest ein über einen Aktuator (4) schaltbares Ventil (5) oder Drosselstelle aufweist, wobei der Aktuator (4) mit der Steuereinrichtung (9) verbunden ist.

5. Orthopädische Einrichtung nach einem der voranstehenden Ansprüche, **dadurch gekennzeichnet, dass** der Steuereinrichtung (9) ein Zeitschaltglied zugeordnet ist, das eine Entriegelung oder Verriegelung nach einem entsprechenden Signal verzögert.

6. Orthopädische Einrichtung nach Anspruch 4, **dadurch gekennzeichnet, dass** ein Dämpfungselement dem Aktuator (4) zugeordnet ist, das eine Entriegelung kontinuierlich durchführt.

7. Orthopädische Einrichtung nach einem der voranstehenden Ansprüche, **dadurch gekennzeichnet, dass** die Steuereinrichtung (9) fernbedienbar ausgebildet ist.

8. Orthopädische Einrichtung nach einem der voranstehenden Ansprüche, **dadurch gekennzeichnet, dass** ein Signalgeber (11, 12) an der Einrichtung angeordnet ist, der ein Öffnen der Verriegelungseinrichtung (3) anzeigt oder ankündigt.

9. Verfahren zur Steuerung einer orthopädischen Einrichtung nach einem der voranstehenden Ansprüche, bei dem die Verriegelungseinrichtung (3) in Abhängigkeit von einer ermittelten Orientierung des Oberteils (1) und/oder Unterteils (2) im Raum entriegelt oder gesperrt wird, **dadurch gekennzeichnet, dass** während einer Streckbewegung des Gelenks bei Überschreiten eines festgelegten Gelenkwinkes eine Beugebewegung verriegelt wird, während eine weitere Streckbewegung weiterhin möglich ist.

10. Verfahren nach Anspruch 9, **dadurch gekennzeichnet, dass** bei einer im Wesentlichen horizontalen Ausrichtung des Unterteils (2) die Verriegelungseinrichtung (3) entriegelt wird.

11. Verfahren nach Anspruch 9 oder 10, **dadurch gekennzeichnet, dass** der Winkel zwischen Oberteil (1) und Unterteil (2) ermittelt und die Verriegelungseinrichtung (3) in Abhängigkeit von dem ermittelten Winkel entriegelt oder gesperrt wird.

12. Verfahren nach einem der Ansprüche 9 bis 11, **dadurch gekennzeichnet, dass** eine in dem Unterteil (2) wirksame Kraft ermittelt und in Abhängigkeit von der ermittelten Kraft die Verriegelungseinrichtung (3) entriegelt oder gesperrt wird

13. Verfahren nach einem der Ansprüche 9 bis 12, **dadurch gekennzeichnet, dass** ausgehend von einem Streckanschlag über einen festgelegten Beugebereich in der Verriegelungseinrichtung (3) ein Dämpfungswiderstand eingestellt wird, der eine gedämpfte Beugung ermöglicht und bei Überscheiten des festgelegten Beugewinkels die Verriegelungseinrichtung (3) gesperrt wird.

14. Verfahren nach einem der Ansprüche 9 bis 13, **dadurch gekennzeichnet, dass** vor dem Entriegeln der Verriegelungseinrichtung (3) ein Warnsignal ausgegeben wird.

## Claims

1. An orthopedic device with a joint, which device has an upper part (1) and a lower part (2) mounted thereon such that it can pivot, wherein upper connection means for attachment to a limb are arranged on the upper part (1), and which device also has a locking device (3) that prevents a flexion movement of the upper part (1) relative to the lower part (2), wherein the locking device (3) is embodied such that it can actively be actuated by the user of the orthopedic device, and a control device (9) is associated with the locking device (3), is connected to at least one sensor (6, 7, 8) attached to the device, and unlocks or blocks the locking device (3) as a function of the sensor signal in an automatic fashion, wherein the locking device (3) is designed as a hydraulic damper, **characterized in that** the hydraulic damper allows a damped flexion from an extension stop to a fixed flexion angle and blocks further flexion once the fixed flexion angle has been reached.

2. The orthopedic device as claimed in claim 1, **characterized in that** the sensor is coupled to the control device (9) and designed as a position sensor (6) for registering the orientation of the upper part (1) and/or the lower part (2) in space, as an angle sensor (8) for registering the angle between the upper part (1) and the lower part (2), as a force sensor (7) for registering a force acting in the orthopedic device and/or as a torque sensor (7) for registering a torque acting in the orthopedic device.

3. The orthopedic device as claimed in claim 1 or 2, **characterized in that** the orthopedic device is designed as an orthotic knee joint or as a prosthetic knee joint.

4. The orthopedic device as claimed in one of the preceding claims, **characterized in that** the locking device (3) is a hydraulic damper, which has at least one valve (5) or restriction that can be switched by an actuator (4), wherein the actuator (4) is connected to the control device (9).

5. The orthopedic device as claimed in one of the preceding claims, **characterized in that** a timing switching element is associated with the control device (9), the former delaying unlocking or locking after an appropriate signal.

6. The orthopedic device as claimed in claim 4, **characterized in that** a damping element that unlocks in a continuous fashion is associated with the actuator (4).

7. The orthopedic device as claimed in one of the preceding claims, **characterized in that** the control device (9) has a design that allows remote controlling.

8. The orthopedic device as claimed in one of the preceding claims, **characterized in that** a signal generator (11, 12) is arranged on the device and displays or announces that the locking device (3) is being opened.

9. A method for controlling an orthopedic device as claimed in one of the preceding claims, wherein the locking device (3) is unlocked or blocked as a function of a detected orientation of the upper part (1) and/or the lower part (2) in space, **characterized in that** a flexion movement is locked during an extension movement of the joint if a fixed joint angle is exceeded, but a further extension movement is still possible.

10. The method as claimed in claim 9, **characterized in that** the locking device (3) is unlocked in the case of a substantially horizontal alignment of the lower part (2).

11. The method as claimed in claim 9 or 10, **characterized in that** the angle between upper part (1) and lower part (2) is detected and the locking device (3) is unlocked or blocked as a function of the detected angle.

12. The method as claimed in one of claims 9 to 11, **characterized in that** a force acting in the lower part (2) is detected and the locking device (3) is unlocked or blocked as a function of the detected force.

13. The method as claimed in one of claims 9 to 12, **characterized in that** a damping resistance is set in the locking device (3) over a fixed flexion range, starting from an extension stop, that allows damped flexion and blocks the locking device (3) if the fixed flexion angle is exceeded.

14. The method as claimed in one of claims 9 to 13, **characterized in that** a warning signal is emitted before the locking device (3) is unlocked.

## Revendications

1. Dispositif orthopédique comprenant une articulation, qui comprend une partie supérieure (1) et une partie inférieure (2) montée sur celle-ci avec possibilité de basculement, dans lequel des moyens de raccordement supérieurs sont agencés sur la partie supérieure (1) pour la fixation sur un membre, ainsi qu'un dispositif de verrouillage (3) qui empêche un mouvement de flexion de la partie supérieure (1) par rapport à la partie inférieure (2), dans lequel le dispositif de verrouillage (3) est conçu pour pouvoir être actionné de manière active par l'utilisateur du dispositif orthopédique, et un dispositif de commande (9) est associé au dispositif de verrouillage (3), qui est relié à au moins un capteur (6, 7, 8), qui est monté sur le système est qui déverrouille ou qui bloque automatiquement le dispositif de verrouillage (3) en fonction du signal du capteur, dans lequel le dispositif de verrouillage (3) est réalisé sous forme d'amortisseur hydraulique,
**caractérisé en ce que** l'amortisseur hydraulique permet une flexion amortie, depuis une butée d'extension jusqu'à un angle de flexion déterminé, et bloque la poursuite de la flexion après avoir atteint l'angle de flexion déterminé.

2. Dispositif orthopédique selon la revendication 1, **caractérisé en ce que** le capteur est réalisé comme un capteur de situation (6) pour détecter l'orientation de la partie supérieure (1) et/ou de la partie inférieure (2) dans l'espace, comme un capteur angulaire (8) pour détecter l'angle entre la partie supérieure (1) et la partie inférieure (2), comme un capteur de force (7) pour détecter une force active dans le système orthopédique et/ou comme un capteur de couple (7) pour détecter un couple actif dans le dispositif orthopédique, et est couplé avec le dispositif de commande (9).

3. Dispositif orthopédique selon la revendication 1 ou 2, **caractérisé en ce que** le dispositif orthopédique est réalisé comme une orthèse d'articulation du genou ou une prothèse d'articulation du genou.

4. Dispositif orthopédique selon l'une des revendications précédentes, **caractérisé en ce que** le dispositif de verrouillage (3) est un amortisseur hydraulique qui comprend au moins une valve (5) ou un étranglement susceptible d'être mis en service via un actionneur (4), ledit actionneur (4) étant relié au dispositif de commande (9).

5. Dispositif orthopédique selon l'une des revendications précédentes, **caractérisé en ce qu'**un circuit à commande temporelle est associé au dispositif de commande (9), lequel retarde un déverrouillage ou un verrouillage après un signal correspondant.

6. Dispositif orthopédique selon la revendication 4, **caractérisé en ce qu'**un élément d'amortissement est associé à l'actionneur (4), qui exécute un déverrouillage en continu.

7. Dispositif orthopédique selon l'une des revendications précédentes, **caractérisé en ce que** le dispositif de commande (9) est réalisé de de manière à pouvoir être télécommandé.

8. Dispositif orthopédique selon l'une des revendications précédentes, **caractérisé en ce qu'**un émetteur de signal (11, 12) est agencé sur le système, qui indique ou qui annoncé une ouverture du dispositif de verrouillage (3).

9. Procédé pour la commande d'un dispositif orthopédique selon l'une des revendications précédentes, dans lequel le dispositif de verrouillage (3) est déverrouillé ou verrouillé en fonction d'une orientation déterminée de la partie supérieure (1) et/ou de la partie inférieure (2) dans l'espace, **caractérisé en ce que** pendant un mouvement d'extension de l'articulation en cas de dépassement d'un angle d'articulation déterminé, on verrouille un mouvement de flexion, alors qu'une poursuite du mouvement d'extension est toujours possible.

10. Procédé selon la revendication 9, **caractérisé en ce que** pour une orientation sensiblement horizontale de la partie inférieure (2) le dispositif de verrouillage (3) est déverrouillé.

11. Procédé selon la revendication 9 ou 10, **caractérisé en ce que** l'angle entre la partie supérieure (1) et la partie inférieure (2) est déterminé et le dispositif de verrouillage (3) est déverrouillé ou verrouillé en fonction de l'angle déterminé.

12. Procédé selon l'une des revendications 9 à 11, **caractérisé en ce que** l'on détermine une force active dans la partie inférieure (2), et le dispositif de verrouillage (3) est déverrouillé ou verrouillé en fonction de la force déterminée.

13. Procédé selon l'une des revendications 9 à 12, **caractérisé en ce que**, en partant d'une butée d'extension et sur une plage de flexion déterminée dans le dispositif de verrouillage (3) on établit une résistance d'amortissement, qui permet une flexion amortie et, en cas de dépassement de l'angle de flexion déterminé, le dispositif de verrouillage (3) est verrouillé.

14. Procédé selon l'une des revendications 9 à 13, **caractérisé en ce qu'**avant le déverrouillage du dispositif de verrouillage (3) on envoie un signal d'avertissement.
